(19) Europäisches Patentamt
European Patent Office
Office européen des brevets

(11) Numéro de publication: **0 106 769**
**B1**

(12) **FASCICULE DE BREVET EUROPÉEN**

(45) Date de publication du fascicule du brevet: **14.01.87**

(51) Int. Cl.[4]: **B 01 J 20/32,** C 12 N 11/08, G 01 N 30/00, B 01 D 15/08

(21) Numéro de dépôt: **83402013.3**

(22) Date de dépôt: **14.10.83**

(54) **Support particulaire greffé en surface, son procédé de préparation et adsorbants pour chromatographie d'affinité incorporant ce support, ainsi que leur utilisation, notamment en biologie.**

(30) Priorité: **15.10.82 FR 8217315**

(43) Date de publication de la demande: **25.04.84 Bulletin 84/17**

(45) Mention de la délivrance du brevet: **14.01.87 Bulletin 87/3**

(84) Etats contractants désignés: **AT BE CH DE FR GB IT LI LU NL SE**

(56) Documents cité:
**DE-A-2 709 094**
**FR-A-2 187 849**
**FR-A-2 476 125**
**US-A-4 111 838**
**US-A-4 280 923**

**POLYMER BULLETIN, vol. 3, 1980, pages 645-653, Springer-Verlag C.G. BEDDOWS et al.: "The use of graft copolymers as enzyme supports. The preparation and use of polyethylene-co-acrylic acid supports"**
**POLYMER SCIENCE U.S.S.R., vol. 22, no. 9, 1980, pages 2150-2161, Pergamon Press Ltd., PL N.A. PLATE et al.: "The effect of the method of fixation of proteolytic enzymes in polymeric hydrogels on the blood-compatibility of modified polymers"**

(73) Titulaire: **COMMISSARIAT A L'ENERGIE ATOMIQUE Etablissement de Caractère Scientifique Technique et Industriel, 31/33, rue de la Fédération, F-75015 Paris (FR)**

(72) Inventeur: **Collin, Claude, 3, rue de Bretagne Val des 4 Pignons, F-78650 Beynes (FR)**
Inventeur: **Duval, Dominique, 21, B1 Saint- Antoine Bât. A, F-78000 Versailles (FR)**
Inventeur: **Kirzenbaum, Mareck, 8, rue Anatole France, F-78350 Jouy- en- Josas (FR)**
Inventeur: **Nicaise, Maryvonne, 7, Allée des Bathes, F-91940 Orsay- lès- Ulis (FR)**
Inventeur: **Dumont, Christophe, Estérel 5 49, rue de Châtenay, F-92160 Antony (FR)**
Inventeur: **Gaussens, Gilbert, 11, rue Jean Brunet, F-92190 Meudon (FR)**
Inventeur: **Morillon, Cécile, 99, rue de Paris, F-91400 Orsay (FR)**

(74) Mandataire: **Ayache, Monique, CABINET PLASSERAUD 84, rue d'Amsterdam, F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 106 769 B1

LIBER, STOCKHOLM 1987

**Description**

L'invention concerne un support pour adsorbants utilisables en chromatographie d'affinité, notamment dans le domaine de la biologie.

Plus précisément, l'invention a pour objet un support particulaire, obtenu à partir de particules d'une matière polymère inerte, qui présente en surface des fonctions chimiques aptes à former des liaisons covalentes avec des ligands utilisables en chromatographie d'affinité, notamment pour la séparation ou l'élimination de substances biologiques.

L'invention a également pour objet un procédé de préparation de ce support.

L'invention a en outre pour objet les adsorbants pour chromatographie d'affinité obtenus par fixation covalente d'un ligand approprié sur ce support.

Enfin, l'invention a pour objet l'utilisation de tels adsorbants, notamment pour la récupération ou l'élimination de substances biologiques, en particulier à partir du sang.

L'utilisation de supports inertes porteurs d'éléments biofonctionnels fixés par des liaisons covalentes s'est généralisée et étendue à un grand nombre d'applications biologiques et biomédicales. En particulier, on a déjà proposé d'utiliser, en chromatographie d'affinité, en tant qu'adsorbants, des supports inertes porteurs de ligands appropriés pour la purification de substrats biologiques ou l'extraction de substances biologiques intéressantes à partir de substrats biologiques tels que notamment le sang. Le pouvoir séparateur de l'adsorbant dépend de la spécificité du ligand fixé sur le support vis-à-vis de la substance à éliminer ou à extraire. Il est à ce propos essentiel que les propriétés physico-chimiques et chimiques du support n'aient pas d'effet défavorable sur l'intéraction entre le ligand et la molécule à fixer.

Compte tenu de ces considérations, on a déjà cherché à exploiter de très nombreux supports présentant des propriétés variées.

Ainsi, il existe actuellement un grand nombre de supports utilisés en chromatographie d'affinité. La plupart se présentent sous forme de gels qui diffèrent suivant la dimension des mailles et la composition chimique des chaînes macromoléculaires qui les constituent (cellulose, dextran, agarose, polyacrylamide, polystyrène, divinyl benzène...).

Par ailleurs, certains supports solides tels que billes de verre, céramique, etc., retenant un ligand spécifique, sont utilisés dans le même but en exploitant le phénomène d'absorption.

Toutefois, aucun des supports proposés jusqu'à présent ne s'est révélé satisfaisant,en particulier pour former des adsorbants utilisables en chromatographie d'affinité sur colonne.

On a pu établir que les différents inconvénients présentés en chromatographie d'affinité sur colonne par les supports de l'art antérieur pouvaient être éliminés en ayant recours à un support particulaire (se présentant sous forme de poudre ou de billes selon la taille des particules), présentant une bonne stabilité dimensionnelle tout en possédant une bonne réactivité chimique, pratiquement limitée à sa surface, vis-à-vis du ligand spécifique de la substance, notamment biologique, à récupérer ou éliminer.

On connaît certes déjà de nombreux supports qui, sous forme particulaire, présentent les propriétés mécaniques requises. Cependant on n'avait pas jusqu'à présent résolu de façon satisfaisante le problème de la fixation du ligand choisi sur un tel support.

L'invention apporte une solution satisfaisante à ce problème en fournissant un support particulaire modifié, qui après fixation covalente d'un ligand approprié, conduit à l'obtention d'un adsorbant efficace et stable pour chromatographie d'affinité sur colonne.

Ce résultat est obtenu grâce à la structure particulière de ce support dans lequel les "greffons" sont répartis en surface et présentent une longueur déterminée de telle sorte qu'il peut fixer des molécules, notamment biologiques, par covalence, tout en conservant leur structure sinon native, du moins encore fonctionnelle, avec une accessibilité de leurs fonctions chimiques réactives suffisante pour réagir efficacement avec le composé à séparer par chromatographie d'affinité sur colonne.

Plus précisément, l'invention a pour objet un support particulaire pour la fabrication d'adsorbants pour chromatographie d'affinité sur colonne, caractérisé en ce qu'il est essentiellement constitué de particules d'une matière polymère de base aptes à être utilisées en chromatographie sur colonne et sur lesquelles sont greffées, essentiellement en surface, des molécules d'au moins un monomère insaturé capable de former un copolymère avec la matière de base et porteur d'au moins une fonction chimique capable de former, ultérieurement, dans des conditions non dénaturantes, une liaison covalente avec le ligand utilisé dans l'adsorption envisagée, la quantité de monomère greffé possédant une fonction chimique accessible par unité de surface (monomère dosé par unité de surface) étant de 10 à 500 µg par $cm^2$ de surface du support et le rapport de la quantité de monomère greffé possédant une fonction chimique accessible à la quantite totale de monomère greffé (monomère dosable/ monomère fixé) étant de 0,05 à 0,5.

De préférence, la quantité de monomère dosé par unité de surface est de 30 à 350 µg par $cm^2$ et le rapport monomère dosable/monomère fixé est de 0,05 à 0,3.

On entend ici par "particules d'une matière polymère de base aptes à être utilisées en chromatographie sur colonne" des particules d'une matière présentant notamment et simultanément, à l'état de particules, les qualités suivantes:

- tenue dimensionnelle sous pression,
- résistance à l'écrasement,
- densité supérieure à l'unité,

- inerties chimique et physico-chimique suffisantes dans les conditions d'utilisation.
- insolubilité dans la plupart des solvants, notamment dans l'eau et les solutions aqueuses,
- taux de gonflement faible dans l'eau et les solutions aqueuses.

Parmi les matières qui, à l'état particulaire, répondent à cette définition on peut citer notamment: le chlorure de polyvinyle, le polystyrène, le poly-chloro-trifluoroéthylène, des polyamides et le polyéthylène, et leurs copolymères présentant les caractéristiques susindiquées.

Suivant la taille de ces particules on parlera de "poudres ou microbilles" (taille inférieure à 1 mm) ou de "billes" (particules pratiquement sphériques ayant des diamètres supérieurs à 1 mm).

Le "greffage en surface" du monomère insaturé conduit à "l'encapsulation" plus ou moins importante de la particule dans une couche de copolymère de la matière de base et du ou des monomère(s) insaturé(s) greffé(s), appelés ciaprès, pour simplifier, "le monomère".

Le monomère greffé doit présenter outre une insaturation, par exemple une double liaison carbone-carbone, capable de former, par greffage, un copolymère avec la matière de base, au moins une fonction chimique capable de former ultérieurement une liaison covalente avec le ligand dont l'utilisation est envisagée, et ceci dans des conditions non dénaturantes pour le ligand, c'est-à-dire des conditions qui ne modifient pas de façon importante l'aptitude du ligand une fois fixé à réagir de façon sélective avec la substance à adsorber.

Le monomère greffé comporte, en tant que fonction chimique répondant à cette définition, par exemple et de façon préférée, au moins une fonction chimique choisie parmi:
- les fonctions acide carboxylique activables par les carbodiimides,
- les fonctions amide activables par le glutaraldéhyde,
- les fonctions amine activables par le glutaraldéhyde,
- les fonctions aldéhyde qu'il n'est pas nécessaire d'activer, et
- les fonctions hydroxyle activables par le bromure de cyanogène.

En tant que monomères insaturés répondant à la définition donnée ci dessus on peut citer:
- l'acide acrylique,
- l'acrylamide,
l'allylamine,
- l'acroléine,
- l'acrylate d'éthylène-glycol et
- le méthacrylate de diméthylaminoéthyle.

Les fonctions chimiques accessibles pour des réactions covalentes ultérieures du monomère greffé peuvent être dosées chimiquement selon des méthodes connues de l'homme du métier, éventuellement après modification (hydrolyse des fonctions amide en fonctions acide par exemple).

Le nombre de fonctions chimiques accessibles ainsi déterminé par dosage permet aisément de calculer, à partir du poids moléculaire du monomère greffe, et connaissant la surface de la matière polymère de base. le poids de monomère greffé possédant une fonction chimique accessible (monomère dosable) par unité de surface du supprt avant greffage.

La quantité totale de monomère fixé est la différence entre le poids $P_1$ du support après greffage et le poids $P_0$ de la matière polymère de base.

L'invention a également pour objet un procédé pour la préparation du support particulaire selon l'invention. Ce procédé utilise, comme source d'énergie, des rayonnements ionisants, en l'absence de tout catalyseur chimique.

Plus précisément, le procédé selon l'invention pour la préparation d'un support particulaire greffé, défini précédemment, est caractérisé en ce qu'il consiste essentiellement à effectuer le greffage radiochimique, sur des particules d'une matière polymère de base choisie, du ou des monomère(s) insaturé(s) choisi(s), en solution dans un solvant non agressif vis à vis des réactifs ou en l'absence de solvant.

Selon ce procédé, on a essentiellement recours à trois types de techniques de greffage radiochimiques connus, à savoir:
- le greffage après préirradiation en présence d'oxygène,
- le greffage après préirradiation en absence d'oxygène,
- le greffage direct sous rayonnements.

On rappellera brièvement ci-après le principe de chacune de ces trois techniques.

1) <u>Greffage après préirradiation en présence d'oxygène</u> (greffage sur polymèresperoxydés):

Cette méthode consiste à peroxyder le polymère de base en l'irradiant en présence d'oxygène puis à effectuer le greffage dans une seconde étape au cours de laquelle on décompose, notamment par la chaleur, les peroxydes macromoléculaires formés, en présence du monomère à greffer.

2) <u>Greffage après préirradiation en absence d'oxygène</u>:

Cette méthode consiste à irradier, dans une première étape, un polymère à l'état vitreux ou cristallisé. Les macroradicaux formés ont une mobilité très réduite et certains d'entre eux restent "piégés" sans se recombiner.

Dans une seconde étape, on laisse diffuser le monomère à greffer dans le polymère ainsi traité; les macroradicaux formés précédemment amorcent la polymérisation et on obtient un copolymère greffé.

3) <u>Greffage direct sous rayonnements</u>:

Cette méthode consiste à utiliser directement les macroradicaux formés lors de la radiolyse du polymère de base pour amorcer la polymérisation du polymère à greffer présent dans le milieu au moment de l'irradiation.

Dans tous les cas, les rayonnements ionisants proviennent avantageusement d'accélérateurs d'électrons ou de sources de cobalt 60.

Selon l'invention, on a établi que des résultats particulièrement satisfaisants sont obtenus lorsque le greffage est effectué:

- sur des particules de polymère de base dont la taille est située dans la gamme d'environ 10 à environ 5 000 µ;
- dans un solvant tel que l'eau, l'acétone ou l'éthanol, purs ou en mélange;
- à une concentration du monomère dans le solvant de 10 à 80 % en poids;
- pendant une durée de 1 à 6,5 heures;
- à une température située dans la gamme allant de la température ambiante à la température d'ébullition du solvant utilisé; et
- avec une dose d'irradiation comprise dans la gamme de 0,3 à 10 Mrad.

Selon un mode préféré de réalisation, le procédé selon l'invention pour la préparation du support particulaire greffé, défini précédemment, est donc caractérisé en ce qu'il consiste essentiellement à effectuer le greffage radiochimique, sur des particules d'une matière polymère de base choisie dont la taille est située dans la gamme d'environ 10 à environ 5 000 µ, du monomère insaturé choisi, en solution à une concentration de 10 à 80 % en poids, dans l'eau, l'acétone ou l'éthanol, purs ou en mélange, pendant une durée de 1 à 6,5 heures, à une température située dans la gamme allant de la température ambiante à la température d'ébullition du solvant utilisé, avec une dose d'irradiation comprise dans la gamme de 0,3 à 10 Mrad.

Le support particulaire greffé en surface selon l'invention, obtenu comme il vient d'être décrit, ou selon tout autre procédé, est stable et peut être conservé tel quel.

Ce support particulaire greffé est avantageusement utilisé pour préparer des adsorbants pour chromatographie, notamment dans le domaine de la biologie.

Dans ce domaine, depuis déjà un certain nombre d'années, on effectue des recherches intensives en vue de mettre au point des dispositifs permettant d'extraire ou d'éliminer, de manière sûre et efficace, différentes substances à partir de matières biologiques, en particulier à partir du sang.

Pour ce faire, un des moyens les plus efficaces envisagés jusqu'à ce jour consiste à adsorber la substance à extraire ou à éliminer sur un adsorbant muni d'une substance apte à réagir de façon spécifique avec ladite substance à extraire ou à éliminer.

Ainsi, on peut par exemple éliminer du sang, in vitro ou par circulation extracorporelle ex vivo, un anticorps, un antigène ou un substrat d'enzyme en faisant passer le sang à purifier sur un adsorbant muni respectivement du l'antigène, anticorps ou enzyme correspondant.

Cette méthode peut également être utilisée, notamment in vitro, pour récupérer à partir du sang des constituants biologiques recherchés, notamment en thérapeutique, tels qu'antigènes ou anticorps.

Le support particulaire greffé en surface selon l'invention convient particulièrement bien à la préparation de tels adsorbants.

En effet, un tel support présente à sa surface un grand nombre de sites réactifs vis-à-vis notamment des ligands de substance biologiques. Comme on peut, en faisant varier notamment la taille des particules, la nature du monomère greffé et la longueur des greffons grâce à un choix approprié des conditions de greffage, régler sur ce support la distance, la densité et la répartition des sites réactifs, il est possible d'y fixer par covalence un très grand nombre de ligands, en particulier des ligands spécifiques de substances biologiques.

La fixation covalente du ligand sur le support particulaire a lieu selon des méthodes connues, telles que décrites par exemple dans Immunochemistry 1, 219-229, (1964), J. of Immunological Methods 11, 129-133 (1976) et Science 195, 302 (1977), après activation des sites réactifs du support. Il est rappelé à ce propos que l'activation des fonctions acide peut se faire au moyen de carbodiimides et celle des fonctions amide et amine au moyen de glutaraldéhyde.

On a en outre établi selon l'invention que les sites réactifs sont rendus plus accessibles pour la fixation covalente du ligand lorsqu'on effectue une hydratation du support, préalable à l'activation. Donc, selon d'invention, ou hydrate de préférence le support avant de procéder à son activation, puis à la fixation covalente du ligand.

La durée de l'hydratation dépend essentiellement de la structure et de la composition du support particulaire greffé.

Cette durée peut être déterminée aisément par l'homme du métier, pour chaque type de support. Par exemple, dans le cas d'un support particulaire greffé à l'acide acrylique, ou à l'acrylamide après hydrolyse des fonctions amide en fonctions acide, on peut déterminer la durée d'hydratation optimum (au bout de laquelle le maximum de fonctions acide sont rendues accessibles) en suivant les variations de pH d'une solution de soude dans laquelle un échantillon du support greffé est mis en suspension.

En tout état de cause, on a pu établir que selon l'invention une durée d'hydratation préalable de 24 heures suffit à rendre accessibles toutes les fonctions utilisables. A ce stade le taux de gonflement, en volume, du support est au maximum de 30 %, le plus souvent de l'ordre de 10 % et le support conserve toutes ses caractéristiques mécaniques désirées pour la chromatographie d'adsorption sur colonne.

L'invention a donc en outre pour objet un adsorbant pour chromatographie d'affinité, caractérisé en ce qu'il résulte de la fixation covalente du ligand spécifique de la substance à adsorber, sur le support particulaire selon l'invention.

L'invention a de plus pour objet un procédé pour la préparation de cet adsorbant pour chromatographie

d'affinité, caractérisé en ce qu'il consiste essentiellement à fixer par covalence le ligand spécifique de la substance à adsorber, sur le support après activation de celui-ci, précédée de préférence d'une hydratation.

Enfin l'invention a pour objet l'utilisation d'un tel adsorbant, en particulier dans le domaine de la biologie, et notamment pour extraire ou éliminer une substance biologique du sang, in vitro ou par circulation extracorporelle ex vivo.

A titre d'exemples, om indiquera a ce propos que:

- pour extraire ou élimimer un antigène du sang on utilisera avantageusement un adsorbant selon l'invention dans lequel le ligand est l'anticorps, par exemple monoclonal, correspondant;
- pour extraire ou éliminer un anticorps du sang on utilisera avantageusement un adsorbant selon l'invention dans lequel le ligand est l'antigène correspondant; et
- pour éliminer un substrat d'enzyme du sang, on utilisera avantageusement un adsorbant selon l'invention dans lequel le ligand est l'enzyme correspondante.

Il va de soi que pour traiter des milieux complexes tels que le sang l'homme du métier devra choisir base de ses connaissances générales en la matière, parmi les nombreux supports particulaires selon l'invention, ceux qui sont compatibles non seulement avec le ligand à fixer, mais encore avec le milieu complexe à traiter avec lequel ils ne devront pas donner de réactions secondaires génantes, en particulier dans le cas de l'épuration du sang par circulation extracorporelle "ex vivo".

L'invention sera mieux comprise à l'aide des exemples non limitatifs qui suivent.

**II Exemples de préparation de supports.**

**Exemple 1**

Poudre de chlorure de polyvinyle greffée par de l'acrylamide, après irradiation en l'absence d'oxygène.

Le support de base est une poudre de chlorure de polyvinyle (commercialisée sous la dénomination PVC Lycovyl GB 1220) dont la granulométrie est comprise entre 100 et 200 microns. Le greffage d'acrylamide est effectué en absence d'oxygène. La poudre est d'abord irradiée en atmosphère d'azote sous rayonnement $\gamma$. Une dose de 0,57 Mrad est appliquée avec un débit de 0,19 Mrad/heure.

La poudre est ensuite mise au contact du monomère en solution dans un mélange eau-acétone (3:5) contenant 15 % d'acrylamide pendant 1h30 à température ambiante, toujours à l'abri de l'oxygène et sous agitation.

La poudre greffée est alors lavée dans un mélange eau-acétone (3:5) et séchée.

Le greffage pondéral qui est défini comme étant égal à

$$\frac{P_1 - P_0}{P_0} \times 100$$

($P_1$ étant le poids final et $P_0$ le poids initial) est de 12,7 %.

L'hydrolyse des fonctions amide accessibles réalisée dans une solution d'acide chlorydrique 3N pendant 3 heures à reflux permet ensuite d'évaluer les fonctions acide carboxylique obtenues et de les doser par la soude. De cette manière le dosage conduit à une évaluation de 2,35 % (en poids) de chaînes dérivées de l'acrylamide présentant un site actif.

**Exemple 2**

Poudre de chlorure de polyvinyle greffée par de l'acrylamide, après irradiation en l'absence d'oxygène.

Le support de base est le même que dans l'exemple 1. Le greffage d'acrylamide est effectué suivant le même procédé et dans les mêmes conditions d'irradiation que dans l'exemple 1. La solution de greffage contient 20 % d'acrylamide et le greffage à la température ambiante dure 1 heure. La poudre lavée et séchée est ainsi greffée à 24 % en poids et 5 % de polyacrylamide actif peuvent être dosés.

**Exemple 3**

Poudre de chlorure de polyvinyle greffée par de l'acide acrylique, après irradiation en l'absence d'oxygène.

Le support de base est le même que dans l'exemple 1. Le greffage d'acide acrylique est effectué en atmosphère d'azote. L'irradiation est faite dans un premier temps, une dose de 0,6 Mrad étant appliquée sous électrons. Le greffage est ensuite réalisé par agitation de la poudre, toujours en atmosphère d'azote, en

suspension dans une solution contenant 10 % d'acide acrylique dans l'eau en présence de 0,15 % d'inhibiteur (sel de Mohr) pendant 1 heure à 85 ± 2°C.

Le greffage est évalué à 3,5 % par dosage des fonctions acide carboxylique par la soude.

**Exemple 4**

Poudre de chlorure de polyvinyle greffée par de l'allylamine, après irradiation en l'absence d'oxygène.

Le support de base est le même que dans l'exemple 1.

L'irradiation et le greffage d'allylamine sont effectués en absence d'oxygène, sous vide. La poudre est préirradiée sous électrons ayant une énergie de 3 MeV. La dose appliquée est de 5 Mrad. Le greffage est réalisé en laissant la poudre au contact d'une solution d'allylamine à une concentration de 50 % dans l'éthanol, sous agitation, pendant 1h à 40 ± 2°C. Les lavages sont réalisés dans l'éthanol.

Le greffage pondéral mesuré est de 6 %.

**Exemple 5**

Billes de polystyrène greffées par de l'acide acrylique, après irradiation en présence d'oxygène.

Le support, sous forme de billes de diamètre 3,2 mm, est constitué de polystyrène. Il est peroxydé par irradiation à l'air, sous rayonnements γ. Une dose de 7,5 Mrad est appliquée à un débit de 0,44 Mrad/heure. Le support est ensuite agit dans une solution contenant en poids 79,6 % d'acide acrylique, 20 % d'eau, 0,4 % de sel de Mohr, pendant 6h30, à 87 ± 2°C, sous atmosphère d'azote.

Le taux de greffage pondéral est de 2,8 %; il est évalué, par dosage, à 0,29 %.

**Exemple 6**

Billes de polystyrène greffées par de l'acrylamide, après irradiation en présence d'oxygène.

Des billes de polystyrène de 3,2mm de diamètre sont greffées par de l'acrylamide dans les conditions suivantes:

La peroxydation du support est réalisée par irradiation, à l'air, sous électrons (3 MeV) avec une dose de 7,5 Mrad.

Après 24 heures, les billes sont plongées dans une solution contenant en poids 59,4 % d'acrylamide, 40 % d'eau, et 0,6 % de sel de Mohr, dans laquelle barbote de l'azote. La température de la solution est de 92 ± 2°C, la durée de greffage est de 6h30.

Le taux de greffage pondéral est de 0,45 %.

**Exemple 7**

Billes de polystyrène greffées par de l'acrylate de butyle et de l'acide acrylique, apres irradiation en presence d'oxygène.

Des billes de polystyrène de 3,2 mm de diamètre sont greffées simultanément, dans les mêmes conditions d'irradiation que celles décrites dans l'exemple 6, par de l'acide acrylique et de l'acrylate de butyle pour augmenter la balance hydrophile/ hydrophobe.

Le greffage est effectué dans une solution contenant en poids, 5 % d'acrylate de butyle, 74,6 % d'acide acrylique, 20 % d'eau et 0,4% de sel de Mohr, à 87 ± 2°C pendant 1h45.

Le taux de greffage pondéral est de 2,4 %.

**Exemple 8**

Billes de polystyrène greffées par de l'acide acrylique, après irradiation en présence d'oxygène.

Des billes de polystyrène de 3,2 mm de diamètre sont greffées par de l'acide acrylique. La méthode utilisée est la peroxydation, l'irradiation du support étant effectuée sous faisceau d'électrons (3 MeV), à l'air, avec une dose de 7,5 Mrad.

Après 24 heures, les billes, plongées dans une solution contenant en poids 79,6 % d'acide acrylique, 20 % d'eau et 0,4 % de sel de Mohr, sont agitées pendant 4 heures à 87 ± 2°C, en maintenant dans la solution, un barbotage d'azote.

Le greffage pondéral est de 2,6 % et le greffage mesuré par dosage desfonctions acide est de 0,12 %.

**Exemple 9**

Poudre de copolymère chlorure de polyvinyle-acétate de vinyle greffée par de l'acide acrylique, après irradiation en présence d'oxygène.

Le support de base est un copolymère, sous forme de microbilles, de chlorure de polyvinyle contenant 10 % d'acétate de vinyle (commercialisé sous la dénomination Lucovyl SA 6001), dont la granulométrie est comprise entre 100 et 200 microns.

Le greffage de l'acide acrylique est effectué après irradiation, à l'air, de la poudre, sous rayonnements $\gamma$, à une dose de 0,3 Mrad dispensée en 1 heure. La solution de greffage, hors de laquelle l'oxygène est chassé par barbotage d'azote, contient, en poids, 9,95 % d'acide acrylique dans 90 % d'eau, en présence de 0,05 % de sel de Mohr. Elle est chauffée à 80 $\pm$ 2°C durant le temps de greffage de 1 heure.

Le taux de greffage pondéral, mesuré par différence de poids, après lavage et séchage est de 3,6 %. Le dosage des fonctionsacide carboxylique correspond à 1,5 % de greffage.

**Exemple 10**

Poudre de copolymère de chlorotrifluoroéthylène et de fluorure de vinylidène greffée par l'acide acrylique, après irradiation en l'absence d'oxygène.

Un support de base constitué de copolymère de chlorotrifluoréthylène et de fluorure de vinylidène (commercialisé sous la dénomination Kel-F) de granulométrie comprise entre 80 et 160 microns, est greffé par de l'acide acrylique dans les conditions suivantes: une préirradiation du support est réalisée en absence d'oxygène, sous vide, sous électrons (3 MeV) avec une dose de 0,87 Mrad. Le greffage est ensuite effectué, sous vide, à 90 $\pm$ 2°C, pendant 1h10 au contact d'une solution de 20 % d'acide acrylique dans l'eau en présence de 0,2 % de sel de Mohr.

Les fonctions acide dosées correspondent à un taux de greffage dosable de 8,9 %.

**Exemple 11**

Poudre de copolymère de chlorure de polyvinyle et d'acétate de vinyle greffée sous vide par de l'acroléine, après irradiation par un faisceau d'électrons.

Le support de base est une poudre de copolymère de chlorure de polyvinyle - acétate de vinyle contenant 10 % d'acétate de vinyle dont la granulométrie est comprise entre 100 et 200 µ. Le greffage d'acroléine est effectué sous vide, après un double dégazage. La poudre est irradiée par un passage sous faisceau d'électrons (énergie: 3 MeV: intensité: 400 µA) à une dose de 2,05 Mrad. Elle est ensuite mise au contact d'une solution d'acroléine se composant de:

50 % d'acroléine
25 % d'éthanol
25 % d'eau

pendant 17 heures à la température embiante. La poudre greffée est lavée à l'éthanol.

Le greffage est évalué par pesée à 5,3 % et par dosage des fonctions aldéhyde à 1,5 %.

**Exemple 12**

Poudre de polyamide greffée par de l'acrylate d'éthylène-glycol, après irradiation sous vide par un faisceau d'électrons.

Le support de base est un polyamide 11 de granulométrie comprise entre 10 et 100 µ.

Le monomère greffé est l'acrylate d'éthylène-glycol. Le greffage est effectué sur la poudre préirradiée sous faisceau d'électrons (E = 3 MeV. I = 400 µA) en absence d'oxygène. sous vide, à une dose de 9 Mrad, en mettant 10 g de poudre en suspension pendant 15 minutes à 75°C dans 100 ml d'une solution contenant 50 % d'acrylate d'éthylèneglycol dans l'eau. en présence de 0,5 % de sel de Mohr. La poudre est lavée à l'eau et séchee.

Le taux de greffage pondéral est de 186 %.

**Exemple 13**

<u>Poudre de polyéthylène greffée par de l'acrylate d'éthylène-glycol. après irradiation sous vide par un faisceau d'électrons.</u>

Le support de base est un polyéthylène (très haut poids moléculaire) de granulométrie comprise entre 10 et 100 µ.

Le monomère greffé est l'acrylate d'éthylène-glycol. Le greffage est effectué par une méthode de préirradiation en absence d'oxygène, sous vide. La poudre de polyéthylène est irradiée sous faisceau d'électrons (E = 3 Mev, I = 400 µA) à une dose de 9 Mrad. Elle est mise ensuite au contact, toujours sous vide, d'une solution de 49,5 % d'acrylate d'éthylène-glycol et de 0,5 % de sel de Mohr dans l'eau. à 75°C pendant 90 minutes, à raison de 10 ml de solution par gramme de poudre.

La poudre greffée est ensuite lavée à l'eau et séchée. Le taux de greffage pondéral est de 110 %.

**Exemple 14**

<u>Poudre de copolymère de chlorure de polyvinyle et de polyacétate de vinyle greffée par le méthacrylate de diméthylamino-éthyle après préirradiation en l'absence d'oxygéné, par des rayons γ.</u>

Le support de base est une poudre de copolymère de chlorure de polyvinyle et de polyacétate de vinyle à 10 % de polyacétate de vinyle (commercialisée sous la dénomination: Lucovyl SA 6001) dont la granulométrie est comprise entre 100 et 200 µ.

Le greffage du monomère. à savoir le méthacrylate de diméthylaminoéthyle (MADAME) de formule

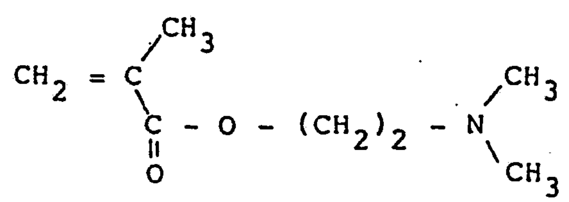

,

est effectué selon une méthode de préirradiation en absence d'oxygène. La poudre est irradiée en atmosphère d'azote sous rayonnement γ à une dose de 1,5 Mrad avec un débit de 0,3 Mrad/h. elle est ensuite mise au contact du monomère en solution contenant 40 % de MADAME, 59,6 % d'eau et 0,4 % d'hydroquinone jouant le rôle d'inhibiteur, à raison de 5 ml de solution par gramme de support. La réaction s'effectue à 30°C pendant une heure, en atmosphère d'azote.

La poudre est ensuite lavée dans des solutions d'eauéthanol et séchée à 50°C. Le taux de greffage pondéral obtenu est de 8,6 %.

**II Exemples de préparation d'adsorbants.**

**Exemple 15**

<u>Fixation d'acides aminés sur un support.</u>

On utilise le support obtenu à l'exemple 1. Après hydratation du support, les fonctions amide sont activées par incubation du support greffé pendant 17h à 37°C dans une solution contenant 6 % de glutaraldéhyde en tampon MES (0,1 M) à pH 7,4, à raison de 25 ml de solution pour un gramme de support greffé.

Le support est mis en suspension à raison de 1 g/10 ml et agité dans une solution d'acide aminé (50 g/l) en tampon MEQ (0,1 M) maintenue à une température de 5°C pendant 5 heures.

Les manipulations sont effectuées à différents pH: 6,5; 7,5; 8,5, avec deux acides aminés différents: la lysine et l'acide glutamique.

Les taux de couplage sont évalués par dosage des fonctions acide carboxylique par la soude 0,1 N.

La validité de ce test est prouvée en confirmant sur témoin l'absence de toute hydrolyse des fonctions amide sur le même support n'ayant pas subi l'activation et le couplage.

Les résultats obtenus sont les suivants:

| | pH | Nombre de moles fixées par gramme | Rendement par rapport au greffage |
|---|---|---|---|
| Lysine | 6,5 | $2,55.10^{-4}$ | 77 % |
| | 7,5 | $1,34.10^{-4}$ | 40,5 % |
| | 8,5 | $2,2\ .10^{-4}$ | 66,7 % |
| Acide glutamique | 6,5 | $0,88.10^{-4}$ | 26,5 % |
| | 7,5 | $1,08.10^{-4}$ | 33 % |
| | 8,5 | $1,03.10^{-4}$ | 31,1 % |

**Exemple 16**

Fixation d'une enzyme (chymotrypsine) sur un support.

On utilise le support obtenu à l'exemple 1. Après hydratation du support, l'activation est effectuée dans des conditions identiques à celles décrites à l'exemple 15. Le couplage de la chymotrypsine est réalisé au pH optimum de 7 dans des conditions équivalentes à celles décrites pour le couplage des acides aminés. La concentration de la solution de chymotrypsine n'est que de 2 g/litre.

Le support subit ensuite 4 lavages:

1er lavage: 17h en tampon MES (0,1 M)

2eme lavage: 7h,en tampon MES (0,1 M) NaCl(IM) à pH 7. 3eme lavage: 41h dans une solution de HCl($10^{-3}$M) NaCl(IM) à pH 3.

4eme lavage: 7h en tampon TRIS (0,01M)$CaCl_2$(0,05 M) à pH 8.

Les résultats sont évalués en:

- poids de chymotrypsine par gramme de support, déduits des mesures de densité optique sur le milieu réactionnel; et
- activités enzymatiques mesurées sur les différentes solutions et sur la poudre.

L'activité enzymatique est exprimée en Unités Internationales (UI) [-on rappelle qu'une Unité Internationale représente le nombre de micromoles de soude ajoutées, nécessaires pour neutraliser les fonctions acide carboxylique libérées par l'hydrolyse de l'ester éthylique de l'acétyltyrosine (ATEE) sous l'action de la chymotrypsine.]

Dans la solution de couplage la concentration en chymotrypsine chute de 1,93 g/l à 1,815 g/l; une quantité de 1,15 mg est donc fixée par gramme support.

Parallèlement l'activité enzymatique de la solution chute de 259 UI à 7,68 UI.

L'activité enzymatique finale mesurée par gramme de support est de 25,8 UI.

**Exemple 17**

Fixation de γ-globulines sur un support.

On utilise le support obtenu à l'exemple 2. Après hydratation du support, les fonctions amide sont activées en agitant la poudre en suspension dans une solution a 2,5 % de glutaraldéhyde en tampon phosphate à pH 7,2, pendant 1 heure à 4°C. Après rinçage de la poudre, on couple des γ-globulines G humaines marquées à l'iode 125, par agitation de la poudre en suspension dans une solution de 1 mg/ml de γ-globulines (γg) en tampon phosphate à 4°C, pendant une nuit. Des lavages intenses en tampon phosphate sont répétés afin d'éliminer les protéines adsorbées.

Dans ces conditions, la fixation de γg est évaluée à 0,6 mg par gramme de support.

**Exemple 18**

Fixation de γ-globulines sur un support.

On utilise le support obtenu à l'exemple 3. L'activation des fonctions acide carboxylique, après hydratation, est réalisée en présence de 1-cyclohexyl-3-(2-morpholino-éthyl)carbodiimide dans une solution tampon MES acide 2-N(morpholino)éthane-sulfonique (0,1 M), à pH 4,75, pendant 1 heure à 20°C. Le support rincé est mis en suspension dans une solution de γ-globulines G humaines (1 mg/ml) et agité une nuit à 4°C. Les lavages sont effectués en tampon MES. La fixation atteint 7 mg de γ-globulines G humaines par gramme de support.

**Exemple 19**

Fixation de γ-globulines sur un support.

On utilise le support obtenu à l'exemple 4. La fixation de γ-globulines G humaines marquées à l'iode 125, après hydratation et activation par le glutaraldéhyde conduit, dans les conditions habituelles, à la fixation de 0,7 mg de γg par gramme de support.

**Exemple 20**

Fixation de γ-globulines sur un support.

On utilise le support obtenu à l'exemple 5.

Les γ-globulines G humaines fixées sur ce support dans des conditions équivalentes à celles décrites dans l'exemple 18 sont de 25 à 35 µg par $cm^2$ de surface du support.

**Exemple 21**

Fixation de γ-globulines sur un support.

On utilise le support obtenu à l'exemple 6.

La fixation de γ-globulines G humaines dans des conditions équivalentes à celles décrites dans l'exemple 17 atteint 20 à 30 µg par $cm^2$ de surface du support.

**Exemple 22**

Fixation de γ-globulines sur un support.

On utilise le support obtenu à l'exemple 7.

La fixation de γ-globulines G humaines dans les conditions habituellement employées pour les fonctions acide carboxylique (exemple 18) est de 45 µg/$cm^2$ de surface du support.

**Exemple 23**

Fixation de γ-globulines sur un support.

On utilise le support obtenu à l'exemple 8.

Le support permet dans les conditions habituelles (exemple 18) la fixation de 80 à 130 µg de γ-globulines G humaines par $cm^2$ de sa surface.

**Exemple 24**

Fixation d'hématies humaines sur un support.

On utilise le support obtenu à l'exemple 9.

La fixation irréversible d'hématies humaines sur ce support est réalisée après hydratation et traitement intermédiaire du support par l'éthylène-imine fixée par le carbodiimide.

Les microbilles greffées sont plongées dans une solution d'éthylène-imine de poids moléculaire 30 000 à 40 000 dans un tampon MES, sous agitation rotative, à raison de 50 mg d'éthylène-imine par gramme dg microbilles. Ce traitement dure 90 minutes à température ambiante. Les microbilles sont ensuite transférées

dans une solution contenant, en concentrations finales, les éléments suivants, pour 0,25 gramme de microbilles/ml de solution:

- polyéthylène-imine: 12,5 mg/ml
- 1-cyclohexyl-3(2-morpholinoéthyl)carbodiimide: 50 mg/ml

L'ensemble est maintenu 48h à température ambiante, sous agitation rotative.

Les microbilles sont rincées à l'eau et traitées à nouveau 4h à température ambiante dans une solution contenant le carbodiimide (50 mg/ml) et du chlorure d'ammonium (1M). Après de nombreux rinçages, elles sont stockées en tampon TRIS (10 mM) à pH 7,4 en présence de $NaN_3$ (0,2 %). Avant de permettre l'adsorption des hématies sur les microbilles, les 2 éléments sont lavés séparément dans un tampon sucrose-acétate (7/3) à 310 mOSM à pH 5 (il est rappelé que 1 OSM est la pression osmotique exercée par une solution aqueuse contenant 1 mole de soluté (molécule ou ion) par litre, séparée de l'eau pure par une membrane imperméable à ce soluté).

Une suspension 50/50 en volume de microbilles est ajoutée goutte à goutte dans une suspension 50/50 en volume de globules rouges, dans les mêmes proportions. Une agitation rotative très légère est appliquée pendant 10 minutes. Un rinçage est ensuite effectué avec le même tampon à pH 7,4. On prépare une solution de glutaraldéhyde (310 mOSM - 1 %) dans un tampon PBS (Phosphate Buffer Saline: solution isotonique tamponnée). Les microbilles sont mises en suspension à raison de 10 % de microbilles dans une solution contenant 0,5 % de glutaraldéhyde. La réaction dure 1 heure à température ambiante. Différents rinçages sont effectués à 4° C:

- glycine (0,1 M)
- tampon PBS suivis d'une incubation d'une heure en solution de glycine. Le rinçage final est effectué en tampon PBS.

Les hématies du milieu réactionnel sont comptées sur cellule de Malassez (lame qui permet de dénombrer les cellules) et évaluées en outre après hémolyse en milieu ammoniacal. On peut ainsi estimer qu'il y a de 8 à $9.10^8$ hématies fixées par gramme de support.

**Exemple 25**

Fixation d'hématies humaines sur support.

On utilise le support obtenu à l'exemple 4 sur lequel on adsorbe des hématies humaines puis on les fixe au glutaraldéhyde. Les conditions opératoires décrites dans l'exemple 24 sont reprises, en évitant le traitement l'éthylène-imine.

La fixation d'hématies est estimée du même ordre de grandeur, soit 8 à $9.10^8$ hématies par gramme de support.

**Exemple 26**

Fixation d'acide glutamique sur un support.

On utilise le support obtenu a l'exemple 10.

L'activation des fonctions acide carboxylique et la fixation d'acide glutamique sont réalisées en un seul temps. La poudre est mise en suspension pendant 17h à 4°C dans une solution contenant l'acide glutamique (50 g/l) et dul-cyclohexyl-3-(2-morpholinoéthyl)carbodi-imide (10 %) en tampon MES (0,1M) à pH 4,75. Pour 1 gramme de support 10 ml de solution sont nécessaires.

Le dosage des fonctions acide carboxyliqué sur le support couplé permet, par rapport au témoin non couplé, d'évaluer un taux de 14 % en poids d'acide glutamique fixé par liaison irréversible.

**Exemple 27**

Fixation de chymotrypsine sur un support.

On utilise le support obtenu à l'exemple 11.

Le couplage de la chymotrypsine est réalisé en agitant 1 gramme de support greffé pendant 20h à 20°C dans 10 ml d'une solution tampon phosphate de potassium (0,05 M à pH 7) contenant 20 mg de chymotrypsine.

La poudre est ensuite lavée abondamment en milieu tampon phosphate (pH 7), puis en milieu tampon phosphate-NaCl (1M) puis dans une solution de HCl($10^{-3}$M)-NaCl(1M) à pH 3. ét enfin en tampon TRIS (0,01 M) -$CaCl_2$ (0,05 M) à pH 8.

L'activité enzymatique de la poudre ainsi obtenue est de 22 UI/gramme de support (l'activité initiale de la chymotrypsine en solution est de 13 UI/mg).

**Exemple 28**

Fixation de β-galactosidase sur un support.

On utilise le support obtenu à l'exemple 12. Les fonctions hydroxyle sont activées par le bromure de cyanogène dans les conditions suivantes: 1 g de support est mis en suspension à température ambiante pendant 15 minutes dans une solution de carbonate de sodium (0,02 M) dont le pH est ajusté à 11 par la soude et contenant 10 % de bromure de cyanogène, à raison de 30 millimoles de bromure de cyanogène pour 1 g de support.

Après rinçage dans une solution de bicarbonate de sodium (0,1 M), la poudre est mise en suspension dans 10 ml d'une solution de β-galactosidase à 2 g/l dans un tampon bicarbonate pH 9,3, pendant 24 heures à 4°C. La poudre est ensuite lavée abondamment et successivement dans 4 solutions:

- $NaHCO_3$ (0,1 M)
- HCl ($10^{-3}$ M)
- NaCl (0,5 M)
- eau distillée.

L'activité enzymatique de la β-galactosidase fixée est mesurée après incubation de la poudre à 37°C pendant 20 minutes en présence d'orthonitrophénol-D-galactopyranoside (ONPG). substrat dont l'orthonitrophénol (ONP) est libéré par hydrolyse. proportionnellement à la quantité d'enzyme présente.

L'activité ainsi mesurée est de 0,84 micromole d'ONP libérée par gramme de support.

**Exemple 29**

Fixation de β-galactosidase sur un support.

On utilise le support obtenu à l'exemple 13.

L'activation des fonctions hydroxyle par le bromure de cyanogène ainsi que le couplage de la β-galactosidase sont realisés dans les conditions décrites dans l'exemple 28.

De même l'activité enzymatique de l'enzyme fixée est mesurée par l'action hydrolytique de l'enzyme sur le substrat ONPG.

L'activité mesurée est de 0,45 micromole d'ONP libérée par gramme de support.

**Exemple 30**

Fixation d'hématies humaines sur un support.

On utilise le support obtenu à l'exemple 14.

L'adsorptionet la fixation des globules rouges sont réalisées directement sur le support, aprés avoir lavé séparément les billes et les globules rouges en tampon sucrose-acétate (7/3) à pH 5.

Une suspension 50/50 en volume de microbilles est ajoutée goutte à goutte dans une suspension 50/50 en globules rouges, dans les mêmes proportions. Une agitation rotative très légère est appliquée pendant 10 minutes. Un rinçage est ensuite effectué avec le même tampon à pH 7,4.

On prépare une solution de glutaraldéhyde: 1 % dans le tampon PBS (solution isotonique tamponnée). Les microbilles sont mises en suspension à raison de 10 % de microbilles dans une solution contenant 0,5 % de glutaraldéhyde. La réaction dure 1 heure à température ambiante. Différents rinçages sont effectués à 4°C:

- glycine (0 1 M)
- tampon PBS

suivis d'une incubation d'une heure en solution glycine. Le rinçage final est effectué en tampon PBS.

Le nombre d'hématies fixées est évalué à 8 à $9.10^8$ par gramme de support (1,8 à $2.10^6$ hématies/$cm^2$).

**III Utilisation des adsorbants selon l'invention: appli cation à l'èpuration immunologique.**

L'épuration immunologique est une application particulière du principe de la chromatographie d'affinité. L'élimination du milieu plasmatique des éléments indésirables (ex. anticorps, antigènes, complexes immunes) est réalisée par mise en contact avec un support qui possède à sa surface les éléments actifs réagissant spécifiquement avec l'élément à éliminer.

Les techniques d'èpuration plasmatique les plus couramment employées sont actuellement les plasmaphereses.

Les éliminations sont dans ce cas non spécifiques avec perte importante de plasma, coûteuses et on observe un certain nombre d'accidents.

La fabrication d'adsorbants obtenus à partir de supports polymères greffés, possédant des caractéristiques mécaniques variées et ayant fixé irréversiblement différentes biomolécules, ouvre une large voie d'application

dans ce domaine.

Quelques uns des adsorbants décrits dans la deuxième partie ont été utilisés pour mettre en évidence leur capacité d'épuration.

A. Epuration "in vitro"

**Exemple 31**

On utilise l'adsorbant obtenu à l'exemple 18. Des lapins (blancs de Bouscat) sont immunisés par injection intradermique de γ-globulines G humaines (de la société Sigma: fraction II de Cohn). Des prélèvements réguliers sont effectués pour le dosage des anticorps et la constitution de "pools d'anticorps" utilisés lors des "épurations in vitro".

Le dosage quantitatif des anticorps est effectué par une méthode radioimmunologique du type sandwich utilisant des γ-globulines G humaines (γGH) radioiodées ($I^{125}$). Selon cette méthode, des γGH fixées sur des particules de cellulose sont mises en contact simultanément avec l'antisérum et le traceur 125I γGH en excès. Les γGH fixées sur cellulose sont en excès pour piéger tous les anticoros L'excès de traceur est éliminé par lavage. La quantité de traceur fixée au support par l'intermédiaire des anticorps et des γGH est proportionnelle à la quantité d'anticorps présente dans l'antisérum. Les résultats comparés à un standard sont exprimés en µg/ml.

Le pool d'anticorps prélevé sur lapin est dilué avec du sérum de lapin normal. A l'aide d'une pompe péristaltique (de type Mastuflex) la solution est perfusée dans l'épurateur: le système fonctionne en circuit fermé; des prélèvements successifs sont effectués pour le dosage des anticorps anti γGH et des protéines totales.

Les conditions opératoires de deux essais et les résultats d'épuration "in vitro" sont indiqués dans le tableau suivant.

| Essai n° | 1 | 2 |
|---|---|---|
| - Surface totale du support ($cm^2$) | 4.200 | 4.200 |
| - Concentration en anti-corps (µg/ml) | 19,7 | 43 |
| - Volume de plasma (ml) | 30 | 50 |
| - Quantité d'anticorps (mg) | 591 | 2.150 |
| - Débit (ml/mn) | 5 | 5 |
| - Temps (mn) | 60 | 60 |
| - % d'épuration | 97 | 75 |
| - Anticorps "épurés" (µg) | 573 | 1.612 |

**Exemple 32**

On utilise l'adsorbant obtenu à l'exemple 20 pour réaliser des essais d'épuration de sérum renfermant des anticorps anti-γ-globulines G humaines, dans des conditions analogues à celles de l'exemple 31.

Les conditions opératoires de deux essais d'épuration "in vitro" et les résultats obtenus sont rassemblés dans le tableau suivant.

| Essai n° | 1 | 2 |
|---|---|---|
| - Surface totale du support ($cm^2$) | 210 | 420 |
| - Conc. en anticorps (µg/ml) | 4,7 | 4,7 |
| - Volume de plasma (ml) | 30 | 30 |
| - Quantité d'anticorps (µg) | 141 | 141 |
| - Débit (ml/mn) | 15 | 15 |
| - Temps (mn) | 60 | 60 |
| - % d'épuration | 10 | 40 |
| - Anticorps "épurés" (µg) | 14 | 56,4 |

**Exemple 33**

On utilise l'adsorbant obtenu à l'exemple 22 pour réaliser un essai d'épuration de sérum renfermant des anticorps anti-γ- globulines G humaines, dans des conditions analogues à celles de l'exemple 31.

Surface totale du support ($cm^2$)............ 630
Conc. en anticorps (µg/ml).................. 11
Volume de plasma (ml)....................... 30
Quantité d'anticorps (µg)................... 330
Débit (ml/mn)............................... 20
Temps (mn).................................. 60
% d'épuration............................... 33
Anticorps "épurés" (µg)..................... 109

**Exemple 34**

On utilise l'adsorbant obtenu à l'exemple 23 pour réaliser un essai d'épuration de sérum renfermant des anticorps anti-γ-globulines G humaines, dans des conditions analogues à celles de l'exemple 31.

Surface totale du support ($cm^2$)............ 210
Concentration en anticorps (µg/ml).......... 16
Volume de plasma (ml)....................... 30
Quantité d'anticorps (µg)................... 480
Débit (ml/mm)............................... 5
Temps (mn).................................. 60
% d'épuration............................... 9
Anticorps "épurés" (µg)..................... 48

**Exemple 35**

On utilise l'adsorbant obtenu à l'exemple 24.

La capacité d'adsorption spécifique de l'antigène A porté par les hématies fixées sur le support a été vérifiée par rapport à l'activité non spécifique vis-à-vis d'un anticorps B. Pour cela les taux d'anticorps anti A et anti B contenus dans un sérum ont été mesurés avant et après incubation d'une heure à 37°C du support dans ce

sérum. On élimine les anticorps anti A présents dans un sang frais humain.

Les activités sont mesurées suivant la méthode décrite par C. ROPARS, A. MULLER, C. HUREL et J. LEBLANC dans Bl. Transf. and Imm., tome XXIV, n° 1(1981)

Le sérum de départ est titré au 256e.

Les conditions de deux essais réalisés et leurs résultats sont indiqués dans le tableau suivant:

| Essai n° | 1 | 2 |
|---|---|---|
| - Poids de support sec (mg) | 320 | 630 |
| - Dilution du sérum | 1/4 | 1/2 |
| - Quantité de sérum (ml) | 2 | 2 |
| - Epuration : anti A | 93 % | 87,6 % |
| - Epuration : anti B | ∿ 0 | ∿ 0 |

Le taux de protéines total est resté pratiquement constant.

**Exemple 36**

On utilise l'adsorbant obtenu à l'exemple 30.

L'épuration in vitro effectuée sur 2 ml de sérum avec 320 mg de support sec, selon la technique décrite dans l'exemple 35, conduit à une épuration spécifique anti A. de 85 % et une épuration non spécifique anti B. de 5 %.

B.**Epuration ""ex vivo".**

**Exemple 37**

On utilise l'adsorbant obtenu à l'exemple 18.

Un shunt artério-veineux (jugulaire-carotide) est préalablement posé sur un lapin immunisé par injection intradermique de γ-globulines G humaines et anesthésié au Nembutal.

Le sang est perfusé directement sur une colonne contenant 25 à 30 g de l'adsorbant. Le débit est de 10 à 15 ml/mn. L,animal est hépariné au préalable et par perfusion au cours de l'opération. La circulation sanguine F extracorporelle dure 1h30. Le taux d'anticorps est de 22,2 µg/ml au départ et de 2,21 µg/ml après épuration. Les protéines totales chutent de 56 mg/ml à 44,8 mg/ml.

L'invention ne se limite aucunement à ceux de ses modes de réalisation et d'application envisagés ou décrits dans les exemples. En particulier, on obtient des résultats satisfaisants en effectuant le greffage direct du monomère sur le support particulaire de base, notamment dans les conditions préférées qui ont été définies précédemment.

**Revendications**

1. Support particulaire pour la fabrication par fixation covalente d'un ligand, d'adsorbants pour chromatographie d'affinité sur colonne, essentiellement constitué de particules d'une matière polymère de base aptes à être utilisées en chromatographie sur colonne et sur lesquelles sont greffées des molécules d'au moins un monomère insaturé, capable de former un copolymère avec la matière de base et porteur d'au moins une fonction chimique capable de former, ultérieurement, dans des conditions non dénaturantes, une liaison covalente avec le ligand utilisé dans l'adsorption envisagée, caractérisé en ce que les molécules de monomère

insaturé sont greffées essentiellement à la surface des particules de matière polymère de base, la quantité de monomère greffé possédant une fonction chimique accessible par unité de surface (monomère dosable par unité de surface) étant de 10 à 500µg par $cm^2$ de surface du support et le rapport de la quantité de monomère greffé possédant une fonction chimique accessible à la quantité totale de monomère greffé (monomère dosable/monomère fixé) étant de 0,05 à 0,5.

2. Support particulaire selon la revendication 1, caractérisé en ce que la quantité de monomère dosable par unité de surface est de 30 à 350µg par $cm^2$ et le rapport monomère dosable/monomère fixé est de 0,05 à 0,3.

3. Support particulaire selon la revendication 1 ou 2, caractérisé en ce que la matière polymère de base est choisie parmi le chlorure de polyvinyle, le polystyréne, le polychlorotrifluoroéthylène, les polyamides et le polyéthylène et leurs copolymères aptes à être utilisés, à l'état de particules, en chromatographie sur colonne.

4. Support particulaire selon l'une des revendications 1 à 3, caractérisé en ce que le monomère porte au moins une fonction acide carboxylique, une fonction amide, une fonction amine, une fonction aldéhyde ou une fonction hydroxyle.

5. Support particulaire selon la revendication 4, caractérisé en ce que le monomère est choisi parmi l'acide acrylique, l'acrylamide, l'allylamine, l'acroléine, l'acrylate d'éhylène-glycol et le méthacrylate de diméthylamino-éthyle.

6. Procédé pour la préparation du support particulaire greffé selon l'une des revendications 1 à 5, caractérisé en ce qu'il consiste essentiellement à effectuer le greffage radiochimique, sur des particules d'une matière polymère de base choisie dont la taille est située dans la gamme d'environ 10 à environ 5000 µ du monomère insaturé choisi, en solution à une concentration de 10 à 80% en poids, dans l'eau, l'acétone ou l'éthanol purs ou en mélange, pendant une durée de 1 à 6,5 heures, à une température située dans la gamme allant de la température ambiante à la température d'ébullition du solvant utilisé, aprés préirradiation par des rayonnements ionisants provenant d'accélérateurs d'électrons ou de sources de cobalt 60, avec une dose d'irradiation comprise dans la gamme de 0.3 à 10 Mrad.

7. Procédé selon la revendication 6, caractérisé en ce que la préirradiation et le greffage radiochimique sont effectués en l'absence d'oxygène.

8. Procédé selon la revendication 6, caractérisé en ce que la préirradiation est effectuée en présence d'oxygène et le greffage radiochimique en l'absence d'oxygène.

9. Adsorbant pour chromatographie d'affinité, caractérisé en ce qu'il résulte de la fixation covalente, sur le support particulaire selon l'une des revendications 1 à 5, d'un ligand spécifique de la substance à adsorber.

10. Procédé pour la préparation de l'adsorbant selon la revendication 9, caractérisé en ce qu'il consiste à fixer par covalence le ligand spécifique de la substance à adsorber, sur le support aprés activation de celui-ci, précédée de préférence d'une hydratation.

11. Utilisation de l'adsorbant selon la revendication 9, en particulier dans le domaine de la biologie, et notamment pour extraire ou éliminer une substance biologique du sang, "in vitro" ou par circulation extracorporelle "ex vivo".

**Patentansprüche**

1. Teilchenförmiger Träger zur Herstellung von Adsorbentien für die Affinitätssäulenchromatographie durch kovalente Bindung eines Liganden, im wesentlichen bestehend aus Teilchen eines polymeren Grundmaterials, die zur Verwendung in der Säulenchromatographie geeignet sind und auf denen Moleküle zumindest eines ungesättigten Monomeren aufgepfropft sind, das zur Bildung eines Copolymeren mit dem Grundmaterial fähig ist und zumindest eine chemische Funktion aufweist, die danach unter nicht denaturierenden Bedingungen eine kovalente Bindung mit dem bei der beabsichtigten Adsorption benutzten Liganden einzugehen fähig ist, dadurch gekennzeichnet, daß die Moleküle des ungesättigten Monomeren im wesentlichen auf der Oberfläche der Teilchen des polymeren Grundmaterials aufgepfropft sind, wobei die Menge des aufgepfropften Monomeren, das eine zugängliche chemische Funktion aufweist, pro Oberflächeneinheit (bestimmbares Monomeres pro Oberflächeneinheit) 10 bis 500 µg pro $cm^2$ der Oberfläche des Trägers beträgt, und das Verhältnis der Menge des eine zugängliche chemische Funktion aufweisenden, gepfropften Monomeren zur Gesamtmenge des gepfropften Monomeren (bestimmbares Monomeres/gebundenes Monomeres) 0,05 bis 0,5 beträgt.

2. Teilchenförmiger Träger gemäß Anspruch 1, dadurch gekennzeichnet, daß die Menge des bestimmbaren Monomeren pro Oberflächeneinheit 30 bis 350 µg pro $cm^2$, und das Verhältnis bestimmbares Monomeres/gebundenes Monomeres 0,05 bis 0,3 betragen.

3. Teilchenförmiger Träger gemäß Anspruch 1 oder 2, dadurch gekennzeichnet, daß das polymere Grundmaterial unter Polyvinylchlorid, Polystyrol, Polychlortrifluorethylen, den Polyamiden und Polyethylen oder deren Copolymeren ausgewählt ist, die im Teilchenzustand zur Verwendung bei der Säulenchromatographie geeignet sind.

4. Träger gemäß einem der Ansprüche 1 bis 3, dadurch gekennzeichnet, daß das Monomere zumindest eine Carbonsäure-, Amid-, Amin-, Aldehyd- oder Hydroxylfunktion aufweist.

5. Teilchenförmiger Träger gemäß Anspruch 4, dadurch gekennzeichnet, daß das Monomere unter Acrylsäure, Acrylamid, Allylamin, Acrolein, Ethylenglycolacrylat und Dimethylaminoethylmethacrylat

ausgewählt ist.

6. Verfahren zur Herstellung des teilchenförmigen, gepfropften Trägers gemäß einem der Ansprüche 1 bis 5, dadurch gekennzeichnet, daß es im wesentlichen darin besteht, daß man die Propfung des ausgewählten ungesättigten Monomeren radiochemisch auf Teilchen eines ausgewählten polymeren Grundmaterials durchführt, deren Größe etwa zwischen 10 bis etwa 5.000 μ liegt, und zwar in Lösung bei einer Konzentration von 10 bis 80 Gew.-% in Wasser, Aceton oder Ethanol oder in deren Gemisch, während 1 bis 6,5 Stunden, bei einer Temperatur von Umgebungstemperatur bis zur Siedetemperatur des benutzten Lösungsmittels, und nach Vorbestrahlung durch ionisierende Strahlen aus Elektronenbeschleunigern oder Cobalt 60-Quellen, bei einer Strahlendosis von 0,3 bis 10 Mrad.

7. Verfahren gemäß Anspruch 6, dadurch gekennzeichnet, daß man die Vorbestrahlung und das radiochemische Propfen in Abwesenheit von Sauerstoff durchführt.

8. Verfahren gemäß Anspruch 6, dadurch gekennzeichnet, daß man die Vorbestrahlung in Gegenwart von Sauerstoff, und die radiochemische Propfung in Abwesenheit von Sauerstoff vornimmt.

9. Adsorbens für die Affinitätschromatographie, dadurch gekennzeichnet, daß es durch kovalente Bindung eines spezifischen Liganden der zu adsorbierenden Substanz auf dem teilchenförmigen Träger gemäß einem der Ansprüche 1 bis 5 hergestellt worden ist.

10. Verfahren zur Herstellung des Adsorbens gemäß Anspruch 9, dadurch gekennzeichnet, daß man den spezifischen Liganden der zu adsorbierenden Substanz an den Träger nach seiner Aktivierung, vorzugsweise nach einer vorhergehenden Hydratisierung, durch kovalente Bindungen bindet.

11. Verwendung des Adsorbens gemäß Anspruch 9, insbesondere auf biologischem Gebiet, vor allem zur Extraktion oder Ausscheidung einer biologischen Substanz des Blutes "in vitro" oder, durch extrakorporelle Zirkulation, "ex vivo".

## Claims

1. A particulate support for the manufacture, by covalently bonding of a ligand, of adsorbents for affinity chromatography on a column, said support being essentially constituted by particles of a base polymeric material adapted for use in column chromatography and on which are grafted molecules of at least one unsaturated monomer capable of forming a copolymer with the base material and bearing at least one chemical function capable of forming, subsequently, under non-denaturing conditions, a covalent bond with the ligand used in the envisaged adsorption, characterized in that the molecules of unsaturated monomer are grafted essentially at the surface of the particules of the base polymeric material, the amount of grafted monomer possessing an accessible chemical function per unit of surface area (estimable monomer per unit of surface area) being from 10 to 500 μg per $cm^2$ of surface area of the support, and the ratio of the amount of grafted monomer possessing an accessible chemical function to the total amount of grafted monomer (estimable monomer/fixed monomer) being from 0.05 to 0.5.

2. A particulate support according to Claim 1, characterized in that the amount of estimable monomer per unit of surface area is 30 to 350 μg per $cm^2$ and the ratio estimable monomer/ fixed monomer is 0.05 to 0.3.

3. A particulate support according to Claim 1 or 2, characterized in that the base polymeric material is selected from among polyvinyl chloride, polystyrene, polychlorotrifluoroethylene, polyamides and polyethylene and their copolymers adapted to be used, in the state of particles, in column chromatography.

4. A particulate support according to anyone of Claims 1 to 3, characterized in that the monomer bears at least a carboxylic acid function, an amide function, an amine function, an aldehyde function or a hydroxyl function.

5. A particulate support according to Claim 4, characterized in that the monomer is selected from among acrylic acid, acrylamide, allylamine, acrolein, ethylene-glycol acrylate and dimethylamino-ethyl methacrylate.

6. A process for the preparation of the grafted particulate support according to anyone of Claims 1 to 5, characterized in that it consists essentially in carrying out the radiochemical grafting, on particles of a selected base polymeric material whose size is in the range from about 10 to about 5000 μ, of the selected unsaturated monomer, in solution at a concentration of 10 to 80 % by weight, in water, acetone or ethanol, pure or in admixture, for a period of 1 to 6.5 hours, at a temperature in the range from room temperature to the boiling temperature of the solvent used, after preirradiation with ionizing radiations from electron accelerators or cobalt 60 sources, with a radiation dose comprised in the range of 0.3 to 10 Mrad.

7. A process according to Claim 6, characterized in that the pre-irradiation and the radiochemical grafting are carried out in the absence of oxygen.

8. A process according to Claim 6, characterized in that the pre-irradiation is carried out in the presence of oxygen and the radiochemical grafting in the absence of oxygen.

9. An adsorbent for affinity chromatography, characterized in that it results from the covalent fixation of a specific ligand of the substance to be adsorbed, to the particulate support according to anyone of Claims 1 to 5.

10. A process for the preparation of the adsorbent according to claim 9, characterized in that it consists in fixing by covalence the specific ligand of the substance to be adsorbed, on the support after activation of the latter, preceded preferably by hydration.

11. Use of the adsorbent according to Claim 9, in particular in the field of biology, and particularly to extract or remove a biological substance from the blood, "in vitro" or by extracorporal circulation "ex vivo".